# EUROPEAN PATENT SPECIFICATION

(11) **EP 2 899 264 B1**
(45) Date of publication and mention of the grant of the patent: **01.08.2018**
(21) Application number: 13838519.0
(22) Date of filing: 11.09.2013
(51) Int. Cl.: C12N 1/20, C12P 7/62

(54) **EMULSION FOR MICROBIAL GROWTH, METHOD FOR GROWING MICROORGANISMS BY USING SAID EMULSION, AND METHOD FOR PRODUCING MICROBIAL METABOLITE**
EMULSION FÜR MIKROBIELLEN WACHSTUM, VERFAHREN ZUM ZÜCHTEN VON MIKROORGANISMEN UNTER VERWENDUNG DIESER EMULSION UND VERFAHREN ZUR HERSTELLUNG EINES MIKROBIELLEN METABOLITEN
ÉMULSION POUR CROISSANCE MICROBIENNE, PROCÉDÉ DE CULTURE DE MICRO-ORGANISMES FAISANT APPEL À LADITE ÉMULSION, ET PROCÉDÉ DE PRODUCTION D'UN MÉTABOLITE MICROBIEN

(30) Priority: 20.09.2012 JP 2012207257
(43) Date of publication of application: 29.07.2015
(73) Proprietor: Kaneka Corporation, Kita-ku Osaka 530-8288 (JP)
(72) Inventor: SATO, Shunsuke, Takasago-shi Hyogo 676-8688 (JP); TAKEMURA, Tomonobu, Tokyo 107-6025 (JP); MATSUMOTO, Keiji, Takasago-shi Hyogo 676-8688 (JP)
(74) Representative: Hoffmann Eitle
(86) International application number: PCT/JP2013/074511
(87) International publication number: WO 2014/045962

(56) References cited:
- EP-A1- 2 418 277
- WO-A1-2011/064167
- JP-A- 2005 185 181
- Anonymous: "What's in raw milk", RAW-MILK-FACTS.COM , 21 June 2012 (2012-06-21), page 5PP, XP002753097, Retrieved from the Internet: URL:http://www.raw-milk-facts.com/what_is_ in_raw_milk.html [retrieved on 2016-01-18]
- HUI LIN TAN ET AL: "Na-caseinate/oil/water systems: Emulsion morphology diagrams", JOURNAL OF COLLOID AND INTERFACE SCIENCE, vol. 381, no. 1, 15 May 2012 (2012-05-15), pages 48-58, XP028501270, ACADEMIC PRESS, NEW YORK, NY, US ISSN: 0021-9797, DOI: 10.1016/J.JCIS.2012.05.033 [retrieved on 2012-05-24]
- CHARLES F BUDDE ET AL: "Growth and polyhydroxybutyrate production byin emulsified plant oil medium", APPLIED MICROBIOLOGY AND BIOTECHNOLOGY, vol. 89, no. 5, 29 January 2011 (2011-01-29), pages 1611-1619, XP019880871, SPRINGER, BERLIN, DE ISSN: 1432-0614, DOI: 10.1007/S00253-011-3102-0
- FUKUI T ET AL: "Efficient production of polyhydroxyalkanoates from plant oils by alcaligenes eutrophus and its recombinant strain", APPLIED MICROBIOLOGY AND BIOTECHNOLOGY, vol. 49, no. 3, 1 March 1998 (1998-03-01), pages 333-336, XP002979648, SPRINGER, DE ISSN: 0175-7598, DOI: 10.1007/S002530051178
- CHING-YEE LOO ET AL: "Biosynthesis and Characterization of Poly(3-hydroxybutyrate-co-3- hydroxyhexanoate) from Palm Oil Products in a Wautersia eutropha Mutant", BIOTECHNOLOGY LETTERS, vol. 27, no. 18, 1 September 2005 (2005-09-01), pages 1405-1410, XP019230966, SPRINGER NETHERLANDS, DORDRECHT ISSN: 1573-6776, DOI: 10.1007/S10529-005-0690-8
- CHAN P L ET AL: "Production of medium-chain-length polyhydroxyalkanoates by Pseudomonas aeruginosa with fatty acids and alternative carbon sources", APPLIED BIOCHEMISTRY AND BIOTECHNOLOGY, vol. 132, no. 1-3, 1 March 2006 (2006-03-01), pages 933-941, XP002688627, HUMANA PRESS, INC, UNITED STATES ISSN: 0273-2289, DOI: 10.1385/ABAB:132:1:933
- BUDDE, C. F. ET AL.: 'Growth and polyhydroxybutyrate production by Ralstonia eutropha in emulsified plant oil medium' APPLIED MICROBIOLOGY AND BIOTECHNOLOGY vol. 89, no. 5, March 2011, pages 1611 - 1619, XP019880871
- TANGNU, S. K., E: 'Emulsification phenomena in salicylic acid fermentation' PROCESS BIOCHEMISTRY vol. 15, no. 6, August 1980, pages 30 - 32, XP008178659
- BANI-LABER, A. ET AL.: 'Efficacy of the Antimicrobial Peptide Nisin in Emulsifying Oil in Water' JOURNAL OF FOOD SCIENCE vol. 65, no. 3, 2000, pages 502 - 506, XP055242481
- MATIAS, F. ET AL.: 'Polyhydroxyalkanoates production by actinobacteria isolated from soil' CANADIAN JOURNAL OF MICROBIOLOGY vol. 55, no. 7, 2009, pages 790 - 800, XP008178662
- KUMAR, T. ET AL.: 'POTENTIAL OF BACILLUS SP. TO PRODUCE POLYHYDROXYBUTYRATE FROM BIOWASTE' JOURNAL OF APPLIED MICROBIOLOGY vol. 106, 2009, pages 2017 - 2023, XP055242483
- XU, Y. ET AL.: 'Microbial biodegradable plastic production from a wheat-based biorefining strategy' PROCESS BIOCHEMISTRY vol. 45, 2010, pages 153 - 163, XP026796452

## Description

### TECHNICAL FIELD

The present invention relates to the technical field of emulsions for microbial growth. The present invention also relates to the technical field of methods for culturing microorganisms using the emulsions for microbial growth. The present invention also relates to the technical field of methods for producing microbial metabolites.

### BACKGROUND ART

As a result of the increasing awareness of environmental problems, food problems, and health and safety issues, the growing orientation towards non-synthetic or natural products, and the like, production of microorganisms and microbial production of materials (e.g., fermentative production and bioconversion) are increasingly gaining importance and significance.

The production of microorganisms and the microbial production of materials require carbon sources for culture, fermentation or the like, which can be suitably assimilated by microorganisms. Representative examples of such carbon sources include carbohydrates, fats and oils (e.g. animal and vegetable fats and oils), and fatty acids (e.g. fatty acids derived from animals or plants).

In cases where carbohydrates such as glucose and sucrose, which are carbon sources having high solubility in water, are used in the production of microorganisms or microbial production of materials, these carbon sources are dissolved in the culture media used for culturing microorganisms, under normal operating conditions, and thus can be readily utilized as carbon sources.

Animal- or plant-derived fatty acids, fats and oils, which are renewable sources, are also carbon sources useful for the production of microorganisms and the microbial production of materials such as polyhydroxyalkanoic acids (PHAs). For example, these days the yield of palm oil that is solid at room temperature is increasing every year due to its high stability and price competitiveness. With this increase, the amount of fatty acids produced as by-products during the refining of fats and/or oils is also increasing every year. There is a need to use these fats, oils and fatty acids, which have low solubility in media, as carbon sources for microorganisms.

However, the most significant problem associated with culturing microorganisms using fats or oils, or fatty acids, which are components of fats and oils, is that they have very low solubility in water. Those having low water solubility form liquid droplets or solid particles in culture media, which can be expected to decrease the microbial assimilation rate. Further, if the melting point of the fat and/or oil and/or fatty acid is higher than the culture temperature, it is considered that the solid particles form aggregates larger than the liquid droplets, causing a further decrease in the assimilation rate. For example, palm oil is solid at about 36°C.

With respect to the water solubility of fatty acids such as lauric acid, myristic acid, palmitic acid, and stearic acid, Non-Patent Literature 1 teaches that even the most soluble lauric acid has a water solubility of 0.1 g/L at most, and acids, such as palmitic acid or stearic acid, aggregate at a concentration of 1 µM or less. These properties cause a decrease in microbial assimilation. In addition, Non-Patent Literature 2, for example, discloses that a PHA was produced by culturing *Aeromonas hydrophila* using lauric acid as a sole carbon source. In this method, the dry cell weight was about 8 g/L, which means that the carbon source could not be suitably utilized. The document also teaches that the difficulty in culture was increased by the fact that lauric acid was solid at the culture temperature.

According to Patent Literature 1, a PHA was produced by culturing *Ralstonia eutropha* (currently classified as *Cupriavidus necator*) using lauric acid or myristic acid as a sole carbon source. In this method, PHA productivity was as low as 1 g/L or less, which means that the carbon source could not be suitably utilized.

According to Non-Patent Literature 3, a PHA was produced by culturing *Escherichia coli* using a fatty acid metal salt such as sodium laurate as a sole carbon source. In this method, sodium laurate was used in an amount of about 2 g/L.

According to Non-Patent Literature 4, a PHA was produced using a fat and/or oil having low solubility and *Ralstonia eutropha* (currently classified as *Cupriavidus necator*) into which a PHBH synthase gene derived from *Aeromonas caviae* was introduced. In this document, palm oil having a melting point higher than the culture temperature (30°C) was used, and the dry cell weight was as low as 4.1 g/L after 72-hour culture, which means that the carbon source could not be suitably utilized.

As a technique that makes efficient use of a carbon source having low solubility, Non-Patent Literature 5 discloses adding sunflower oil to a culture medium through a ceramic membrane to finely disperse the oil having low solubility in the medium. The document describes the production of a surfactant or PHA using bacteria of the genus *Pseudomonas.* This technique characteristically uses a membrane, and thus the range of application of the technique is unfortunately limited to carbon sources that have high melting points and are liquid at the culture temperature. In addition, since the dispersion is not an emulsion, the dispersed particles can be expected to fuse together, failing to maintain the finely dispersed state.

According to Patent Literature 2, lauric acid, myristic acid, palmitic acid, stearic acid, oleic acid, or linoleic acid was emulsified to improve the fermentative productivity of L-threonine or L-lysine from the fatty acids. This document discloses a method for culture using lauric acid, myristic acid, palmitic acid, or stearic acid having a melting point higher than the culture temperature. However, there is a case where although amino acid productivity was improved, the number of viable cells was reduced, and thus this method is not suitable to accumulate PHA in cells. Further, this method allows the content ratio of oleic acid to palmitic acid, for example, to be reduced so that a decrease in productivity occurs, which limits the range of application of the method. In addition, the fatty acid content in the fatty acid emulsion was as low as 20 g/L, making it difficult to apply the method to industrial production.

According to Non-Patent Literature 6, gum arabic (GA) was added as an emulsifier for emulsifying palm oil to shorten the lag time at the start of culture. However, the effect of GA in this document is unclear, and this document teaches that SDS and Triton X-100, which are fatty acid-based emulsifiers, inhibit growth of microorganisms. Moreover, the document does not disclose an increase in the rate of microbial growth by the use of the emulsified carbon source. Further, it describes that from an economical standpoint, emulsification using GA is difficult to apply to industrial production.

According to Patent Literature 3, an emulsion of a vegetable fat and/or oil or a fatty acid methyl ester was used in a microbial fermentation process. This document discloses a method of preparing an emulsified composition using palm oil or rapeseed oil. This method requires addition of 1% by weight or more of an ethoxylated fatty acid or fatty acid alcohol as an emulsifier to a fatty acid, which implies economical difficulties. In addition, the document does not describe the actual use as a raw material for microorganisms, nor whether microorganisms can utilize the ethoxylated emulsifier.

As a culture technique that controls the melting point of the carbon source, Patent Literature 4 discloses an efficient production of PHA using a fat and/or oil, a fatty acid, or a mixture thereof as a raw material. This document discloses a method of reducing the melting point of the carbon source by the use of a mixture of multiple types of fatty acids, fats or oils. However, when the melting point of the carbon source was higher than the culture temperature, a greater temperature difference between the melting point of the carbon source and the culture temperature was found to result in a greater reduction in microbial assimilation. Thus, the usable carbon sources are limited. As described above, there are still problems in achieving the production of microorganisms or the microbial production of materials in an industrially efficient manner by suitably using fatty acids, fats and/or oils, which have low solubility in media, as carbon source.

### CITATION LIST

### PATENT LITERATURE

Patent Literature 1: U.S. Patent Publication No. 20020086377A1
Patent Literature 2: JP-A 2008-167746
Patent Literature 3: PCT Publication No. WO 2000/071676
Patent Literature 4: PCT Publication No. WO 2010/113497

### NON PATENT LITERATURE

Non-Patent Literature 1: Henrik Vorum, et al., Biochmicaet Biophysica Acta., 1126: 135-142 (1992)
Non-Patent Literature 2: Lee S., et al., Biotechnol. Bioeng., 67: 240-244 (2000)
Non-Patent Literature 3: Regina V., et al., FEMS MICROBIOLOGY LETTERS, 111-117 (2000)
Non-Patent Literature 4: T. Fukui, et al., Appl. Microbiol. Biotechnol., 49: 333-336 (1998)
Non-Patent Literature 5: Anuj Dhariwal, et al., Bioprocess Biosyst. Eng., 31: 401-409 (2008)
Non-Patent Literature 6: Charles F. Budde, et al., Appl. Microbiol. Biotechnol., 89: 1611-1619 (2011)

### SUMMARY OF INVENTION

### TECHNICAL PROBLEM

The present invention aims to provide methods for producing materials from microorganisms in an industrially efficient manner by using, as carbon source, lipids having high ascending melting points and low solubility in media at the temperature for culture of the microorganisms. The present invention also aims to provide methods for producing microbial metabolites in an industrially efficient manner by using the lipids as carbon source.

### SOLUTION TO PROBLEM

The present inventors examined methods for producing materials from microorganisms in an industrially efficient manner by using lipids having low solubility in media as carbon source. As a result, they found that emulsions containing a lipid, a phosphate, and a protein are very stable. Further, they found that the use of the emulsions allows the microorganisms to suitably assimilate lipids having high ascending melting points and low solubility in media at the temperature for culture of the microorganisms. Still further, they found that the use of the emulsions enables culture of microorganisms and production of microbial metabolites in an industrially efficient manner, thus accomplishing the present invention.

The present invention relates to a method for culturing a microorganism as well as to the use of an emulsion for culturing a microorganism as defined in the claims. The emulsion for microbial growth comprises a lipid, a phosphate, and a protein.

The protein is a lactoprotein.

The lipid preferably has a water solubility at 25°C of 10 g/L or less.

The lipid has a free fatty acid content of 30% or more.

The lipid content in the emulsion for microbial growth is 3 to 70%.

The present invention also relates to a method for culturing a microorganism, the method including adding the emulsion for microbial growth to a medium.

The lipid in the emulsion for microbial growth preferably has an ascending melting point higher by at least 2°C than a temperature for culturing the microorganism.

The microorganism is preferably a bacterium.

The bacterium is preferably of the genus *Cupriavidus.*

The bacterium of the genus *Cupriavidus* is preferably *Cupriavidus necator* or a recombinant thereof.

The present invention also relates to a method for producing a microbial metabolite, the method including culturing a microorganism by the above method.

The microbial metabolite is preferably a polyhydroxyalkanoic acid.

The polyhydroxyalkanoic acid is preferably poly(3-hydroxybutyrate-co-3-hydroxyhexanoate) .

### ADVANTAGEOUS EFFECTS OF INVENTION

The present invention enables culture of microorganisms and production of microbial metabolites in an industrially efficient manner by using lipids having low water solubility at the temperature for culture of the microorganisms as carbon sources that can be suitably assimilated by the microorganisms.

### DESCRIPTION OF EMBODIMENTS

The present invention is described in detail below. The emulsion for microbial growth of the present invention contains a lipid, a phosphate, and a protein. The term "emulsification" as used herein refers to a state in which one of two or more substances that are mutually insoluble is dispersed in the other substance (s), for example, by stirring these substances. Such combinations of substances include mixtures of water and fats and/or oils, mixtures of water and fatty acids, and mixtures of water, fats and/or oils, and fatty acids. Moreover, according to the definition of the term "emulsification" as used herein, the two or more substances are not necessarily of the same phase. For example, the definition of the term "emulsification" includes a state in which the continuous phase is liquid and the dispersed phase is solid.

The term "lipid" as used herein refers to a fatty acid, a fat and/or oil, and/or a mixture thereof. The type of lipid depends on the type of microorganism used, the type of microbial metabolite to be produced by the microorganism, and the culture conditions (e.g. medium components, pH, and culture temperature) . Any type of lipid can be used as long as it can be assimilated by the microorganism and has low water solubility, and as long as the lipid has a free fatty acid content as defined below.

Examples of fatty acids include free fatty acids as well as fatty acid salts and fatty acid esters. These may be used alone or in admixture. Any fatty acid can be suitably used in the present invention as long as the microorganism used has a metabolic pathway for the fatty acid.

Examples of fats and oils include glycerides such as triglycerides, diglycerides, and monoglycerides, and these may be used alone or in admixture. Any fat and/or oil can be suitably used in the present invention as long as the microorganism used has a metabolic pathway for the fat and/or oil.

Moreover, the lipid is preferably derived from a non-petroleum source from an environmental burden standpoint. Typically, animal and vegetable oils and lipids derived from animal or vegetable oils are preferred. In view of the impact on food problems, it is more preferred to use non-edible fats, oils and/or fatty acids.

Examples of animal and vegetable oils include tallow, lard, milk fat, fish oil, soybean oil, rapeseed oil, sunflower oil, olive oil, sesame oil, canola oil, cottonseed oil, peanut oil, tung oil, rice oil, rice oil, safflower oil, coconut oil, palm oil, palm kernel oil, shea butter, sal butter, illipe butter, cacao butter, jatropha oil, algae-derived fats and oils, partially refined products of these fats and oils, fractionated oils, hardened oils, and interesterified oils, as well as their components such as fatty acids, fatty acid salts, and fatty acid esters. These fats and oils may be used alone or in combination of two or more.

In view of avoiding competition with food, it is preferred to use a component produced as a by-product in a process of refining the fat and/or oil. Examples of by-products of refining include fatty acid salts formed in alkali refining processes, distillation residues formed in steam refining processes, and distillation residues formed in deodorizing processes. Examples of fatty acid salts include fatty acid sodium, fatty acid potassium, fatty acid calcium, and fatty acid magnesium salts. The distillation residue may include, in addition to the fatty acids as the main components, monoglycerides and diglycerides.

The lipid preferably has a water solubility at 25°C of 10 g/L or less. The lipid may suitably have a water solubility at 25°C of 1 g/L or less. Further, the lipid may suitably have a water solubility at 25°C of even 0.1 g/L or less.

The lipid preferably has a melting point higher by at least 2°C than the temperature during microbial culture. This is because the present invention can exert a higher dispersibility-improving effect on such lipids whose dispersibility in media is usually poor. The melting point of the lipid is more preferably higher by at least 5°C, still more preferably higher by at least 10°C than the culture temperature . For example, the lipid may suitably have a melting point of 32°C or higher. The lipid may also suitably have a melting point of 35°C or higher. Further, the lipid may suitably have a melting point of even 36°C or higher, 39°C or higher, 40°C or higher, 42°C or higher, or 44°C or higher. Yet, the dispersibility-improving effect can be exerted even on lipids that have any melting point lower than the culture temperature but have low solubility in media. Of course, it is also possible to concurrently use a lipid having a melting point lower than the culture temperature and/or a lipid having a water solubility at 25°C of more than 10 g/L. The present invention enables lipids having melting points higher than the culture temperature and low solubility in media to be suitably assimilated by microorganisms, by emulsifying the lipids to increase the specific surface area.

The lipid has a high free fatty acid content because such a form can be easily used by microorganisms. The lipid more specifically has a free fatty acid content of 30% or more, further more preferably 50% or more, and particularly preferably 70% or more. Moreover, the lipid may contain other components as needed.

The protein is selected from lactoproteins and salts thereof. Examples of lactoproteins include casein, sodium caseinate, and whey.

The protein is added at a concentration of 0.01 to 10% by weight, preferably 0.05 to 5% by weight, more preferably 0.1 to 1% by weight based on the amount of the lipid.

In general, the following emulsifiers are added to emulsify fats and oils: for example, glycerides such as monoglycerides and diglycerides, and organic acid monoglycerides such as acetic acid monoglycerides, lactic acid monoglycerides, citric acid monoglycerides, succinic acid monoglycerides, and diacetyl monoglycerides; polyglycerides, polyglycerol condensed ricinoleic acid ester, sucrose fatty acid esters, sorbitan fatty acid esters, propylene glycol fatty acid esters, lecithin, fractionated lecithin, enzyme-treated lecithin, saponin, polysaccharides such as gum arabic and xanthane gum, and proteins such as sodium caseinate, soy proteins, and partially degraded gluten, and the like. In the present invention, however, it has been found that the addition of these emulsifiers is not effective and that a very stable emulsion can be achieved by a combination of a protein and a phosphate.

Suitable examples of phosphates include normal phosphates such as disodium hydrogen phosphate and potassium dihydrogen phosphate, pyrophosphates such as sodium pyrophosphate, metaphosphates such as sodium hexametaphosphate, and polyphosphates such as sodium polyphosphate. The combined use of a phosphate and a protein is expected to prevent protein aggregation and to form a protein network, thus producing an emulsification stabilizing effect.

The emulsion of the present disclosure characteristically has greatly improved emulsion stability owing to the use of a protein and a phosphate. Moreover, the emulsion may contain an additional emulsifier as needed. Examples of emulsifiers that can be additionally used include monoglycerides, diglycerides, lecithin, and saponin.

The emulsion is preferably prepared as an oil-in-water emulsion before being used in microbial culture. The thus prepared emulsion has good dispersibility in the medium and can be suitably assimilated by the microorganism.

In view of emulsion stability, the lipid content in the emulsion previously prepared before the microbial culture is 3% to 70%. Meanwhile, the commercial production of microorganisms or microbial production of materials frequently involves the intermittent or continuous addition of the lipid to the fermenter during the culture period. In such cases, if the concentration of the lipid in the medium additionally added is low, the amount of culture fluid will be increased so that productivity can be impaired. Thus, the lipid content in the emulsion previously prepared is more preferably 10% to 65%, still more preferably 20% to 65%, and particularly preferably 40% to 65%. Of course, an emulsion having a lipid content of 60% or more can be very effectively used as a carbon source.

The emulsion may be prepared by any conventionally known method for preparing an emulsion, such as by a method using a stirrer with a propeller or the like, or an in-line mixer such as a static mixer; a method using a combination of dispersion discs for emulsification; and a method of performing pre-emulsification in advance, followed by using a homogenizing device such as TK, ultra mixer, Cleamix, colloid mil, high pressure homogenizer, microfluidizer, Ultimaizer, nanomizer, Emulder, etc. Such homogenizing treatment may be carried out two or more times. Ultrasonic emulsifying devices may also be used.

The oil-in-water emulsion containing as an oil phase a lipid having low solubility in a medium may be utilized in any manner for culture. For example, the oil-in-water emulsion may be previously prepared and stored in a tank or the like before being added to the medium; or an aqueous phase and an oil phase respectively from separate feedstock tanks may be fed into the same line for in-line emulsification, followed by adding the emulsion to the medium.

Microorganisms can be cultured using media containing such emulsions for microbial growth, and microbial metabolites can also be produced using the same. Examples of such microbial metabolites include polyhydroxyalkanoic acids (PHAs), alcohols (e.g. ethanol, butanol, propanol, and 1, 4-butanediol), carboxylic acids (e.g. lactic acid, succinic acid, and adipic acid), amino acids (e.g. glutamine, lysine, and threonine), lipids (e.g. docosahexaenoic acid and eicosapentaenoic acid), proteins, and antibiotics.

PHAs are biodegradable thermoplastic polyesters that are produced and accumulated as an energy storage material in cells of many types of microorganisms. Today, more attention is being paid to non-petroleum derived plastics due to the increasing awareness of environmental issues, and among these, in particular, PHAs produced by microorganisms and accumulated in their cells are expected to have only a small adverse effect on the ecosystem because they can be incorporated into the natural carbon cycle process, and thus practical use of PHAs is desired. For this reason, PHAs are preferred examples of materials to be produced according to the present invention.

The type of microorganisms used for the production of microorganisms or the production of microbial metabolites in the present invention is not particularly limited as long as they can assimilate lauric acid, myristic acid, palmitic acid, oleic acid, fats, oils, or the like, and can produce metabolites. Microorganisms isolated from nature, microorganisms obtained by genetic engineering, and the like may be suitably used. Examples of microorganisms include microbes, archaea, bacteria, yeasts, molds, and actinomycetes.

Specific preferred examples of microorganisms include those of the genera *Escherichia* (such as *Escherichia coli*), *Cupriavidus* (such as *Cupriavidus necator*), *Alcaligenes* (such as *Alcaligenes latas*), *Pseudomonas* (such as *Pseudomonasputida, Pseudomonas fluorescens, Pseudomonas aeruginosa, Pseudomonas resinovorans,* and *Pseudomonas oleovorans*), *Bacillus* (such as *Bacillus megaterium*), *Azotobacter, Nocardia, Aeromonas* (such as *Aeromonas caviae* and *Aeromonas hydrophila*), Ralstonia, Wautersia, and Comamonas (Microbiological Reviews, 54(4), 450-472 (1990)).

In addition to the above microorganisms and the like, recombinants that have been altered to artificially produce a PHA by introducing a PHA synthase gene or the like by genetic engineering techniques may be used when producing PHAs as microbial metabolite. For example, cells altered to artificially produce a PHA can be obtained by suitably using microorganisms of the genera *Cupriavidus, Alcaligenes, Pseudomonas, Bacillus, Azotobacter, Nocardia, Aeromonas, Ralstonia, Wautersia, Comamonas,* and the like; gram-negative bacteria of the genus *Escherichia* and the like; gram-positive bacteria of the genus *Bacillus* and the like; and yeasts of the genera *Saccharomyces, Yarrowia, Candida,* and the like.

### Examples of microorganisms of the genus Candida include Candida maltosa.

Among PHAs, PHBH can be produced using microorganisms that inherently produce PHBH, such as *Aeromonas caviae* or *Aeromonas hydrophila,* or microbial cells that have been altered to artificially produce PHBH by introducing a PHA synthase gene or the like into microorganisms that do not inherently produce PHBH by genetic engineering techniques. The host microorganism into which the gene is introduced may suitably be, for example, *Cupriavidus necator.* Moreover, examples of PHA synthase genes include PHA synthase genes derived from *Aeromonas caviae, Aeromonas hydrophila, Chromobacterium* sp., and the genus *Rhdococcus,* and mutants thereof. Examples of such mutants include nucleic acid sequences that encode PHA synthases modified by deletion, addition, insertion, or substitution of at least one amino acid residue.

The type of PHA is not particularly limited as long as it is produced by a microorganism. The PHA is preferably a polymer of a component selected from C4-C16 3-hydroxyalkanoic acids, or a copolymer of two or more components selected from C4-C16 3-hydroxyalkanoic acids. Examples include poly(3-hydroxybutyrate) (PHB) which is a homopolymer of a C4 3-hydroxyalkanoic acid, poly(3-hydroxybutyrate-co-3-hydroxyhexanoate) (PHBH) which is a copolymer of C4 and C6 3-hydroxyalkanoic acids, poly(3-hydroxybutyrate-co-3-hydroxyvalerate) (PHBV) which is a copolymer of C4 and C5 3-hydroxyalkanoic acids, and polymers of C4-C14 3-hydroxyalkanoic acids.

The culture method is not limited to particular culture conditions (e.g., medium composition, carbon source adding method, culture scale, aeration and stirring conditions, culture temperature, and culture period) as long as it includes adding a carbon source to a medium to culture. Yet, the culture method more preferably includes continuously or intermittently adding a carbon source to a medium. It should be noted that the medium used needs to be in a state that can be stirred in a fermenter. As long as this condition is satisfied, the medium may be a liquid medium, a medium that is separated into two phases, or a suspension medium.

PHAs can be accumulated in microorganisms by the culture methods described above, and then can be recovered from the cells using well known methods. For example, the following method may be used. After culture, the cells are separated from the culture fluid using a centrifuge or the like, and the cells are washed with distilled water and methanol or the like and then dried. A PHA is extracted from the dried cells using an organic solvent such as chloroform. Cell debris are removed by filtration or the like from the solution containing the PHA, and a poor solvent such as methanol or hexane is added to the filtrate to precipitate the PHA. Then, the supernatant is removed by filtration or centrifugation, followed by drying. In this way, the PHA can be recovered.

The term "melting point" as used herein refers to an ascending melting point. The ascending melting point can be measured as follows.
(1) An end of a capillary tube open at both ends and having an internal diameter of 1 mm, an outer diameter of 2 mm or less, and a length of 50 to 80 mm is immersed in a completely melted sample and filled with the sample to a height of 10 mm. Then, the sample is immediately solidified with ice chips or the like.
(2) The sample is tested after being left at 10°C or lower for 24 hours or being left on ice for 1 hour.
(3) The capillary tube is set in an ascending melting point measuring device (EX-871A available from ELEX SCIENTIFIC).
(4) The capillary tube is immersed in a container filled with water having a temperature lower by about 20°C than the expected melting point, and the lower end of a thermometer is placed at a depth of 30 mm below the water surface.
(5) The water in the container is heated in such a manner that the water temperature increases at an initial rate of 2°C/min, while being stirred by an appropriate method.
(6) Once the temperature reaches a temperature lower by about 10°C than the expected melting point, the water is heated in such a manner that the water temperature increases at a rate of 0.5°C/min.
(7) The temperature at which the sample starts to ascend in the capillary tube is defined as the ascending melting point.

In the present invention, improvement in assimilation means an increase in the consumption of the carbon source per hour and a consequent increase in the yield of microbial cells or the yield of a material produced by the microorganism. Moreover, in the present invention, carbon sources may be mixed before being added to the medium, or may be separately added and mixed in the medium.

The yield of microbial cells may be measured by conventionally known methods, such as optical density method or dry cell weight measurement method. The yield of a material produced by the microorganism may be measured by conventionally known methods, such as GC or HPLC. The amount of PHA accumulated in the cells can be measured after extraction from the cultured cells using an organic solvent such as chloroform and drying in accordance with the method of Kato et al. (Appl. MicroBiol. Biotechnol., vol. 45, p. 363 (1996); Bull. Chem. Soc., vol. 69, p. 515 (1996)).

### EXAMPLES

The present invention is more specifically described below by reference to examples. The present invention, however, is not limited to these examples.

Carbon sources used in the examples, including palm fatty acid distillate (PFAD), palm kernel fatty acid distillate (PKFAD), crude palm oil (CPO), and crude palm kernel oil (CPKO), are available from MALAYSIAN BIOTECHNOLOGY CORPORATION SDN BHD. Table 1 shows the amounts of fats and oils (including TG, DG, and MG) and free fatty acids (FFA) in the carbon sources, and fatty acid compositions and melting points thereof.

**[Table 1]**

| LIPID | PFAD | PKFAD | CP0 | CPK0 |
|---|---|---|---|---|
| FFA subtotal | 95.0 | 84.5 | 5.9 | 0.9 |
| C8:0 | 0.0 | 2.9 | 0.0 | 4.0 |
| C10:0 | 0.0 | 2.9 | 0.0 | 3.6 |
| C12:0 | 0.2 | 44.2 | 0.1 | 48.5 |
| C14:0 | 1.2 | 16.2 | 1.0 | 15.9 |
| C16:0 | 47.6 | 13.0 | 44.2 | 8.0 |
| cis-C16:1 | 0.3 | 0.0 | 0.2 | 0.0 |
| C18:0 | 4.3 | 2.4 | 4.5 | 2.2 |
| cis-C18:1 | 35.7 | 15.5 | 38.2 | 15.2 |
| cis-C18:2 | 9.7 | 2.6 | 10.7 | 2.5 |
| cis-C18:3 | 0.4 | 0.0 | 0.3 | 0.0 |
| C20:0 | 0.4 | 0.0 | 0.4 | 0.0 |
| Melting point (°C) | 43.8 | 23.7 | 37.5 | 27.2 |

### (Examples 1 to 13 and Comparative Examples 1 and 2) Preparation of emulsions of various carbon sources

Emulsions were prepared using the materials and amounts shown in Table 2. A procedure for preparing the emulsions is described below. A lipid and water were weighed out. After water was heated to 60°C, a phosphate and a protein were dissolved in the water. After dissolution, the solution was mixed with the lipid, and the mixture was pre-emulsified using a homo mixer (Laboratory Mixer Emulsifier available from Silverson Machines, Inc.) at a stirring rate of 2500 rpm.

The resulting pre-emulsified liquid was further emulsified using a high pressure homogenizer (PANDA2K model available from GEA Niro Soavi) at a pressure of 10 bar, whereby an emulsion was obtained. As for emulsion stability of the thus prepared emulsions, the emulsified liquids were stirred at 60°C for 10 minutes, and then after the stirring was stopped, the emulsions were visually observed for phase separation. In Table 2, "Good" indicates that the emulsion showed no separation, and "Poor" indicates that the emulsion quickly started to separate into the oil phase and the aqueous phase.

**[Table 2]**

| | Oil phase | | | | Aqueous phase | | | | | | Emulsion stability |
|---|---|---|---|---|---|---|---|---|---|---|---|
| | PFAD | PKFAD | CPO | CPKO | Water | Sodium polyphosphate (Calgon 1D) | Sodium hexametaphosphate | Disodium hydrogen phosphate dodecahydrate | Sodium caseinate | Whey | |
| Example1 | 500g | | | | 500g | | | 2g/kg | 3g/kg | | Good |
| Example2 | 500g | | | | 500g | | | 15g/kg | 3g/kg | | Good |
| Example 3 | 500g | | | | 500g | | | 15g/kg | 5g/kg | | Good |
| Example 4 | 600g | | | | 400g | | | 2g/kg | 3g/kg | | Good |
| Example 5 | 600g | | | | 400g | | | 15g/kg | 5g/kg | | Good |
| Example 6 | 600g | | | | 400g | | | 15g/kg | | 5g/kg | Good |
| Comparative Exampte1 | 600g | | | | 400g | | | 15g/kg | | | Poor |
| Comparative Example2 | 600g | | | | 400g | | | | 5g/kg | | Poor |
| Example 7 | 600g | | | | 400g | 2g/kg | | | 3g/kg | | Good |
| Example 8 | 600g | | | | 400g | 15g/kg | | | 3g/kg | | Good |
| Example 9 | 600g | | | | 400g | | 2g/kg | | 3g/kg | | Good |
| Example1 0 | 600g | | | | 400g | | 15g/kg | | 3g/kg | | Good |
| Example1 1 | | 600g | | | 400g | 15g/kg | | | 3g/kg | | Good |
| Exampte12 | | | 600g | | 400g | | 15g/kg | | 3g/kg | | Good |
| Example13 | | | | 600g | 400g | | | 15g/kg | 3g/kg | | Good |

As shown in Table 2, the emulsified liquids containing a lipid, a phosphate, and a protein exhibited high stability, whereas the emulsified liquids containing a phosphate alone or a protein alone exhibited very poor emulsion stability. It is noted that the amount (g/kg) of phosphate added and the amount (g/kg) of protein added in Table 2 are each the amount added per kg of lipid, and that these components were prepared by being dissolved in the aqueous phase.

### (Examples 14 and 15) Culture of Escherichia coli

A microorganism was cultured using the emulsion of Example 5 or 11 in Table 2 as a carbon source. The microorganism used was *E.coli* HB101 (available from Takara Bio Inc.).

The preculture medium used was LB medium (10 g/L Bacto-tryptone, 5 g/L Bacto-yeast extract, 5 g/L NaCl). A growth test was carried out using M9 medium (6 g/L Na₂HPO₄, 3 g/L KH₂PO₄, 0.5 g/L NaCl, 1 g/L NH₄Cl, 1 mM MgSO₄, 0.001 w/v% thiamine, 0.1 mM CaCl₂).

A glycerol stock of *E. coli* HB101 was inoculated into the preculture medium, and cultured at 37°C for 12 hours for preculture. Next, the preculture fluid was inoculated at 2 v/v% into a 500-ml Sakaguchi flask containing 50 ml of the M9 medium containing the emulsion, followed by shake culture at 37°C for 96 hours. The emulsion separately prepared was added at once to the Sakaguchi flask. The concentration of the carbon source was set to 0.5 w/v%. After culture, the cells were recovered by centrifugation, washed with methanol, and lyophilized. Then, the dry cell weight was measured. Table 3 shows the results.

### (Comparative Examples 3 and 4)

The same procedure as in Examples 14 and 15 was carried out, except that non-emulsified PFAD or PKFAD was used as a carbon source instead of the emulsion for microbial growth. Table 3 shows the results.

**[Table 3]**

| | Carbon source | Dry cell weight (g/L) |
|---|---|---|
| Example 14 | Example 5 | 3.19 |
| Example 15 | Example 11 | 3.01 |
| Comparative Example 3 | PFAD | 1.14 |
| Comparative Example 4 | PKFAD | 2.18 |

### (Examples 16 to 19) PHA production using Candida maltosa

The emulsion of Example 5, 11, 12, or 13 in Table 2 was used as a carbon source for PHA production. The microorganism used was *Candida maltosa* AHU-71 pARR-149/171NSx2-phbB (see PCT Publication No. WO 2005/085415).

The preculture medium used was YNB medium (0.67 w/v% yeast nitrogen base without amino acid, 2 w/v% glucose). The PHA production medium used was M2 medium (12.75 g/L (NH₄)₂SO₄, 1.56 g/L KH₂PO₄, 0.33 g/L K₂HPO₄·3H₂O, 0.08 g/L KCl, 0.5 g/L NaCl, 0.41 g/L MgSO₄·7H₂O, 0.4 g/L Ca (NO₃)₂·4H₂O, 0.01 g/L FeCl₃·4H₂O) supplemented with 0.45 ml/L of a trace metal salt solution (0.1 N hydrochloric acid containing 1 g/L FeS·₄·7H₂O, 8 g/L ZnSO₄·7H₂O, 6.4 g/L MnSO₄·4H₂O, and 0.8 g/L CuSO₄·5H₂O dissolved therein) .

A glycerol stock (500 µl) of *Candida maltosa* AHU-71 pARR-149/171NSx2-phbB was inoculated into a 500-ml Sakaguchi flask containing 50 ml of the preculture medium. Then, the strain was cultured at a culture temperature of 30°C for 20 hours. Next, the culture fluid was inoculated at 10 v/v% into a 2-L Sakaguchi flask containing 300 ml of the PHA production medium, followed by shake culture at 30°C for 48 hours. The solidified carbon source was prepared by being directly added dropwise to the M9 medium. Moreover, the emulsion was separately prepared and added at once to the Sakaguchi flask. Culture was carried out at a carbon source concentration of 2.0 w/v%. After culture, the cells were recovered by centrifugation, washed with methanol, and lyophilized. Then, the dry cell weight was measured.

Chloroform (100 ml) was added to the obtained dry cells (about 1 g), and the mixture was stirred at room temperature for 24 hours to extract a PHA from the cells. The cell residue was removed by filtration, and the filtrate was concentrated in an evaporator to a total volume of about 30 ml, followed by gradual addition of about 90 ml of hexane. Then, the mixture was left for 1 hour with gentle stirring. The PHA thus precipitated was separated by filtration, and then vacuum dried at 50°C for 3 hours. The weight of the dried PHA was measured, and the polymer content in the cells was then calculated. Table 4 shows the dry cell weights and PHA yields.

### (Comparative Examples 5 to 8)

The same procedure as in Examples 16 to 19 was carried out, except that non-emulsified PFAD, PKFAD, CPO, or CPKO was used as a carbon source instead of the emulsion for microbial growth. Table 4 shows the results.

**[Table 4]**

| | Carbon source | Dry cell weight (g/L) | PHA content (wt%) | PHA yield (g/L) |
|---|---|---|---|---|
| Example 16 | Example 5 | 11.1 | 42.2 | 4.68 |
| Example 17 | Eaxmple 11 | 11.6 | 41.5 | 4.81 |
| Example 18 | Example 12 | 10.4 | 37.7 | 3.92 |
| Example 19 | Example 13 | 10.8 | 41.3 | 4.46 |
| Comaparative Example 5 | PFAD | 5.22 | 31.1 | 1.62 |
| Comparative Example 6 | PKFAD | 8.13 | 35.9 | 2.92 |
| Comparative Example 7 | CPO | 6.37 | 36.4 | 2.32 |
| Comparative Example 8 | CPKO | 7.62 | 40.8 | 3.11 |

The monomer composition of the produced PHAs was analyzed by gas chromatography as follows. A mixture (2 ml) of sulfuric acid and methanol (15:85) and chloroform (2 ml) were added to the dried PHA (20 mg), and the system was hermetically sealed, and then heated at 100°C for 140 min, thereby obtaining a methyl ester as a PHA degraded product. After cooling, sodium hydrogen carbonate (1.5 g) was gradually added thereto for neutralization, and the mixture was left until carbon dioxide was no longer evolved. After adding diisopropyl ether (4 ml) and sufficiently mixing, the mixture was centrifuged, and the monomer unit composition of the PHA degraded product in the supernatant was analyzed by capillary gas chromatography. The gas chromatograph used was GC-17A available from Shimadzu Corporation, and the capillary column used was NEUTRA BOND-1 (column length 25 m, column internal diameter 0.25 mm, liquid film thickness 0.4 µm) available from GL Sciences Inc. Helium was used as the carrier gas, with a column inlet pressure of 100 kPa, and the sample was injected in an amount of 1 µl. The temperature conditions were as follows: the temperature was increased at a rate of 8°C/min until an initial temperature of 100°C to 200°C, and further increased at a rate of 30°C/min until 200°C to 290°C. As a result of analysis under the above conditions, the product was determined to be a PHA (PHBH) formed from C4 and C6 3-hydroxyalkanoic acid monomers.

### (Examples 20 to 23) PHA production using Cupriavidus necator

A PHA was produced using the emulsion of Example 5, 11, 12, or 13 in Table 2 as a carbon source. The microorganism used was *Cupriavidus necator* KNK-005 (see U.S. Patent No. 7,384,766).

The composition of the seed culture medium used was as follows: 1 w/v% meat-extract, 1 w/v% Bacto-tryptone, 0.2 w/v% yeast extract, 0.9 w/v% Na₂HPO₄·12H₂O, and 0.15 w/v% KH₂PO₄ (pH 6.8).

The composition of the preculture medium used was as follows: 1.1 w/v% Na₂HPO₄·12H₂O, 0.19 w/v% KH₂PO₄, 1.29 w/v% (NH₄)₂SO₄, 0.1 w/v% MgSO₄·7H₂O, 2.5 w/v% palm olein oil, and 0.5 v/v% trace metal salt solution (0.1 N hydrochloric acid containing 1.6 w/v% FeCl₃·6H₂O, 1 w/v% CaCl₂·2H₂O, 0.02 w/v% CoCl₂·6H₂O, 0.016 w/v% CuSO₄·5H₂O, and 0.012 w/v% NiCl₂·6H₂O dissolved therein). Palm olein oil was added at once at a concentration of 10 g/L as a carbon source.

The composition of the PHA production medium used was as follows: 0.385 w/v% Na₂HPO₄·12H₂O, 0.067 w/v% KH₂PO₄, 0.291 w/v% (NH₄)₂SO₄, 0.1 w/v% MgSO₄·7H₂O, and 0.5 v/v% trace metal salt solution (0.1 N hydrochloric acid containing 1. 6 w/v% FeCl₃·6H₂O, 1 w/v% CaCl₂·2H₂O, 0.02 w/v% CoCl₂·6H₂O, 0.016 w/v% CuSO₄·5H₂O, and 0.012 w/v% NiCl₂·6H₂O dissolved therein).

First, a glycerol stock (50 µl) of KNK-005 was inoculated into the seed culture medium (10 ml) and cultured for 24 hours for seed culture. Next, the seed culture fluid was inoculated at 1.0 v/v% into a 3-L jar fermenter (MDL-300 model available from B. E. MARUBISHI Co., Ltd.) containing 1.8 L of the preculture medium. Preculture was carried out by culturing for 28 hours under the following operating conditions: culture temperature 28 °C, stirring rate 500 rpm, aeration rate 1.8 L/min, and pH controlled within the range of 6.7 to 6.8. The pH was controlled using a 14% aqueous ammonium hydroxide solution.

Next, the preculture fluid was inoculated at 5. 0 v/v% into a 5-L jar fermenter (MDS-U50 model available from B. E. MARUBISHI Co., Ltd.) containing 2.5 L of the PHA production medium. The operating conditions were as follows: culture temperature 28 °C, stirring rate 400 rpm, aeration rate 2.1 L/min, and pH controlled within the range of 6.7 to 6.8. The pH was controlled using a 14% aqueous ammonium hydroxide solution. The emulsion was intermittently added. Culture was carried out for 64 hours. After culture, the cells were recovered by centrifugation, washed with methanol, and lyophilized. Then, the dry cell weight was measured.

Chloroform (100 ml) was added to the obtained dry cells (1 g), and the mixture was stirred at room temperature for 24 hours to extract a PHA from the cells. The cell residue was removed by filtration, and the filtrate was concentrated in an evaporator to a total volume of 30 ml, followed by gradual addition of 90 ml of hexane. Then, the mixture was left for 1 hour with gentle stirring. The PHA thus precipitated was separated by filtration, and then vacuum dried at 50°C for 3 hours. The weight of the dried PHA was measured, and the polymer content in the cells was then calculated. Table 5 shows the dry cell weights and PHA yields.

### (Comparative Examples 9 to 12)

The same procedure as in Examples 20 to 23 was carried out, except that non-emulsified PFAD, PKFAD, CPO, or CPKO was used as a carbon source instead of the emulsion for microbial growth. Table 5 shows the results.

**[Table 5]**

| | Carbon source | Dry cell weight (g/L) | PHA content (wt%) | PHA yield (g/L) |
|---|---|---|---|---|
| Example 20 | Example 5 | 152.9 | 77.7 | 118.8 |
| Example 21 | Eaxmple 11 | 155.6 | 78.3 | 121.8 |
| Example 22 | Example 12 | 149.1 | 76.2 | 113.6 |
| Example 23 | Example 13 | 150.7 | 76.5 | 115.3 |
| Comaparative Example 9 | PFAD | 71.4 | 45.1 | 32.2 |
| Comparative Example 10 | PKFAD | 133.7 | 72.4 | 96.8 |
| Comparative Example 11 | CPO | 102.0 | 62.8 | 64.1 |
| Comparative Example 12 | CPKO | 128.7 | 70.5 | 90.7 |

The monomer composition of the produced PHAs was analyzed in the same manner as in Examples 16 to 19 and Comparative Examples 5 to 8. As a result of the analysis, the product was determined to be a PHA (PHBH) formed from C4 and C6 3-hydroxyalkanoic acid monomers.

The above results demonstrate that the use of the emulsion of the present invention achieves better assimilation than the use of a non-emulsified lipid.

## Claims

1. A method for culturing a microorganism, the method comprising
adding an emulsion for microbial growth comprising a lipid, a phosphate, and a protein to a medium,
wherein the lipid has a free fatty acid content of 30% or more, and
wherein the lipid content in the emulsion for microbial growth is 3 to 70%,
wherein the protein is a lactoprotein, and wherein the lactoprotein is added at a concentration of 0.01 to 10% by weight based on the amount of the lipid.

2. Use of an emulsion for culturing a microorganism, the emulsion comprising a lipid, a phosphate, and a protein,
wherein the lipid has a free fatty acid content of 30% or more, and
wherein the lipid content in the emulsion for microbial growth is 3 to 70%,
wherein the protein is a lactoprotein, and wherein the lactoprotein is added at a concentration of 0.01 to 10% by weight based on the amount of the lipid.

3. Method or use according to claim 1 or 2,
wherein the lipid has a water solubility at 25°C of 10 g/L or less.

4. Method or use according to any one of claims 1 to 3,
wherein the lipid in the emulsion for microbial growth has an ascending melting point higher by at least 2°C than a temperature for culturing the microorganism.

5. Method or use according to any one of claims 1 to 4,
wherein the microorganism is a bacterium.

6. Method or use according to claim 5,
wherein the bacterium is of the genus *Cupriavidus.*

7. Method or use according to claim 6,
wherein the bacterium of the genus *Cupriavidus* is *Cupriavidus necator* or a recombinant thereof.

8. A method for producing a microbial metabolite, the method comprising
culturing a microorganism by the method as defined in any one of claims 1 and 3 to 7.

9. The method according to claim 8,
wherein the microbial metabolite is a polyhydroxyalkanoic acid.

10. The method according to claim 9,
wherein the polyhydroxyalkanoic acid is poly(3-hydroxybutyrate-co-3-hydroxyhexanoate) .

## Patentansprüche

1. Verfahren zum Kultivieren eines Mikroorganismus, wobei das Verfahren umfasst:
Zugeben einer Emulsion für mikrobielles Wachstum, die ein Lipid, ein Phosphat und ein Protein umfasst, zu einem Medium,
wobei das Lipid einen freien Fettsäuregehalt von 30 % oder mehr aufweist und
wobei der Lipidgehalt in der Emulsion für mikrobielles Wachstum 3 bis 70 % beträgt,
wobei das Protein ein Laktoprotein ist und wobei das Laktoprotein bei einer Konzentration von 0,01 bis 10 Gew.% basierend auf der Menge des Lipids zugegeben wird.

2. Verwendung einer Emulsion zum Kultivieren eines Mikroorganismus,
wobei die Emulsion ein Lipid, ein Phosphat und ein Protein umfasst,
wobei das Lipid einen freien Fettsäuregehalt von 30 % oder mehr aufweist, und
wobei der Lipidgehalt in der Emulsion für mikrobielles Wachstum 3 bis 70 % beträgt,
wobei das Protein ein Laktoprotein ist und wobei das Laktoprotein bei einer Konzentration von 0,01 bis 10 Gew.% basierend auf der Menge des Lipids zugegeben wird.

3. Verfahren oder Verwendung gemäß Anspruch 1 oder 2,
wobei das Lipid eine Wasserlöslichkeit bei 25°C von 10 g/L oder weniger aufweist.

4. Verfahren oder Verwendung gemäß einem der Ansprüche 1 bis 3,
wobei das Lipid in der Emulsion zum mikrobiellen Wachstum einen aufsteigenden Schmelzpunkt aufweist, der um mindestens 2°C höher als die Temperatur zum Kultivieren des Mikroorganismus ist.

5. Verfahren oder Verwendung gemäß einem der Ansprüche 1 bis 4,
wobei der Mikroorganismus ein Bakterium ist.

6. Verfahren oder Verwendung gemäß Anspruch 5,
wobei das Bakterium eines der Gattung *Cupriavidus* ist.

7. Verfahren oder Verwendung gemäß Anspruch 6, wobei das Bakterium der Gattung *Cupriavidus Cupriavidus necator* oder ein Rekombinantes davon ist.

8. Verfahren zum Herstellen eines mikrobiellen Metabolits, wobei das Verfahren umfasst:
Kultivieren eines Mikroorganismus durch das Verfahren gemäß einem der Ansprüche 1 und 3 bis 7.

9. Verfahren gemäß Anspruch 8,
wobei das mikrobielle Metabolit eine Polyhydroxyalkansäure ist.

10. Verfahren gemäß Anspruch 9,
wobei die Polyhydroxyalkansäure Poly(3-hydroxybutyrat-co-3-hydroxyhexanoat) ist.

## Revendications

1. Procédé de culture d'un micro-organisme, le procédé comprenant
l'ajout d'une émulsion pour croissance microbienne comprenant un lipide, un phosphate, et une protéine à un milieu,
dans lequel le lipide présente une teneur en acides gras libres de 30 % ou plus, et dans lequel la teneur en lipide dans l'émulsion pour croissance microbienne est de 3 à 70 %,
dans lequel la protéine est une lactoprotéine, et dans lequel la lactoprotéine est ajoutée à une concentration de 0,01 à 10 % en poids sur la base de la quantité du lipide.

2. Utilisation d'une émulsion pour cultiver un micro-organisme, l'émulsion comprenant un lipide, un phosphate, et une protéine,
dans laquelle le lipide présente une teneur en acides gras libres de 30 % ou plus, et
dans laquelle la teneur en lipide dans l'émulsion pour croissance microbienne est de 3 à 70 %,
dans laquelle la protéine est une lactoprotéine, et dans laquelle la lactoprotéine est ajoutée à une concentration de 0,01 à 10 % en poids sur la base de la quantité du lipide.

3. Procédé ou utilisation selon la revendication 1 ou 2,
où le lipide présente une solubilité dans l'eau à 25 °C de 10 g/l ou moins.

4. Procédé ou utilisation selon l'une quelconque des revendications 1 à 3,
où le lipide dans l'émulsion pour croissance microbienne présente un point de fusion ascendant supérieur d'au moins 2 °C à une température pour cultiver le micro-organisme.

5. Procédé ou utilisation selon l'une quelconque des revendications 1 à 4,
où le micro-organisme est une bactérie.

6. Procédé ou utilisation selon la revendication 5,
où la bactérie est du genre *Cupriavidus.*

7. Procédé ou utilisation selon la revendication 6,
où la bactérie du genre *Cupriavidus* est *Cupriavidus necator* ou un recombinant de celle-ci.

8. Procédé de production d'un métabolite microbien, le procédé comprenant
la mise en culture d'un micro-organisme par l'intermédiaire du procédé tel que défini selon l'une quelconque des revendications 1 et 3 à 7.

9. Procédé selon la revendication 8,
dans lequel le métabolite microbien est un polyacide hydroxyalcanoïque.

10. Procédé selon la revendication 9,
dans lequel le polyacide hydroxyalcanoïque est un poly(3-hydroxybutyrate-co-3-hydroxyhexanoate).
